# EUROPEAN PATENT APPLICATION

(11) **EP 4 586 192 A1**
(43) Date of publication of application: **16.07.2025**
(21) Application number: 25150865.1
(22) Date of filing: 09.01.2025
(51) Int. Cl.: G06T 7/00, A61B 1/00, A61B 5/00

(54) **IMAGE PROCESSING DEVICE AND OPERATION METHOD OF IMAGE PROCESSING DEVICE**

(30) Priority: 12.01.2024 JP 2024003022
(71) Applicant: FUJIFILM Corporation, Tokyo 106-8620 (JP); Fujita Academy, Toyoake-shi, Aichi 470-1192 (JP)
(72) Inventor: TERAMURA, Yuichi, Kaisei-machi, Ashigarakami-gun, Kanagawa (JP); OTAKA, Yohei, Toyoake, Aichi 470-1192 (JP); SHIBATA, Seiko, Toyoake, Aichi 470-1192 (JP)
(74) Representative: HGF

(57) **Abstract**

The present invention provides an image processing device and an operation method of an image processing device capable of preventing oversight during an examination or reducing a burden on a user in a video endoscopic examination of swallowing.

An image acquisition unit acquires an examination image. A first frame classification unit classifies each frame of the examination image into any one of a swallowing-in-progress frame in which swallowing is in progress or a swallowing-not-in-progress frame in which the swallowing is not in progress. A swallowing block generation unit integrates, in a case in which a temporal interval between the swallowing-in-progress frames temporally adjacent to each other is equal to or less than a first threshold value, at least the adjacent swallowing-in-progress frames as a swallowing block.

## Description

### BACKGROUND OF THE INVENTION

### 1. Field of the Invention

The present invention relates to an image processing device that analyzes an image obtained by a video endoscopic examination of swallowing, and an operation method of an image processing device.

### 2. Description of the Related Art

Since dysphagia occurs in association with aging or a nervous system disease, the importance of an examination on a swallowing function has been increasing in recent years in an aging society. In the examination on the swallowing function, it is desired to specify a pathological condition of aspiration and to perform appropriate treatment or prevention of dysphagia. Therefore, a video endoscopic examination of swallowing (VE) has been established as an evaluation method for dysphagia (swallowing function evaluation examination) (for example, JP2022-179222A and JP2022-179218A).

### SUMMARY OF THE INVENTION

In the video endoscopic examination of swallowing, a doctor who is a user observes an image of swallowing movement obtained through an endoscope and evaluates the movement. Therefore, since it is necessary to observe a large number of acquired images, oversight may occur during the examination. Reviewing a long video after the examination is a burden on the user. Therefore, there has been a demand for prevention of oversight during the examination or reduction of the burden on the user.

JP2022-179222A discloses a system that determines whether the swallowing is in progress or the swallowing is not in progress for each image from an examination image. JP2022-179222A also discloses that deep learning is used to determine whether the swallowing is in progress or the swallowing is not in progress. However, in the determination of only one image, an image similar to a swallowing-in-progress image may be obtained even in a time zone in which the swallowing is not actually in progress, or an image similar to a swallowing-not-in-progress image may be obtained even in a time zone in which the swallowing is actually in progress, so that it has been required to perform swallowing determination with high accuracy in consideration of such similar images.

In addition, JP2022-179218A discloses a method of detecting a blur amount, magnitude of an image difference, and a movement amount of a feature point. However, a value similar to a swallowing-in-progress value may be obtained even in a time zone in which swallowing is not actually in progress, or a value similar to a swallowing-not-in-progress value may be obtained even in a time zone in which the swallowing is actually in progress, and so that it has been required to perform swallowing determination with high accuracy in consideration of such similar values.

In JP2022-179222A, frames acquired for a certain period from the examination image for which it is determined that the swallowing is in progress are determined that the swallowing is in progress, and in JP2022-179218A, an initial stage (start time point) and a final stage (end time point) of the swallowing in which the movement occurs are detected. However, in such methods as disclosed in JP2022-179222A and JP2019-195052A, it is difficult to detect the entire swallowing motion.

An object of the present invention is to provide an image processing device and an operation method of an image processing device capable of preventing oversight during an examination or reducing a burden on a user in a video endoscopic examination of swallowing.

An aspect of the present invention relates to an image processing device comprising: a control processor, in which the control processor acquires an examination image, classifies each frame of the examination image into any one of a swallowing-in-progress frame in which swallowing is in progress or a swallowing-not-in-progress frame in which the swallowing is not in progress, and integrates, in a case in which a temporal interval between the swallowing-in-progress frames temporally adjacent to each other is equal to or less than a first threshold value, at least the adjacent swallowing-in-progress frames as a swallowing block.

It is preferable that the control processor determines that the swallowing-in-progress frame of which a temporal width is equal to or less than a second threshold value or equal to or more than a third threshold value is not the swallowing-in-progress frame, or determines that the swallowing block of which a temporal width is equal to or less than the second threshold value or equal to or more than the third threshold value is not the swallowing block.

Another aspect of the present invention relates to an image processing device comprising: a control processor, in which the control processor acquires an examination image, classifies each frame of the examination image into any one of a plurality of swallowing motion-specific frames in accordance with swallowing motion or a swallowing-not-in-progress frame in which the swallowing is not in progress, and integrates, in a case in which a temporal interval between the swallowing motion-specific frames temporally adjacent to each other is equal to or less than a first threshold value, at least the adjacent swallowing motion-specific frames as a motion-specific swallowing block.

It is preferable that the control processor determines that the swallowing motion-specific frame of which a temporal width is equal to or less than a second threshold value or equal to or more than a third threshold value is not the swallowing motion-specific frame, or determines that the motion-specific swallowing block of which a temporal width is equal to or less than the second threshold value or equal to or more than the third threshold value is not the motion-specific swallowing block. It is preferable that the control processor determines, based on an expression pattern including the swallowing motion-specific frame or the motion-specific swallowing block, whether the expression pattern indicates that the swallowing is in progress or that the swallowing is not in progress.

It is preferable that the swallowing motion-specific frame includes a pre-swallowing frame, a swallowing-in-progress frame, or a post-swallowing frame. It is preferable that the pre-swallowing frame includes a pre-swallowing blur frame, an epiglottis inversion frame, or a food intake frame. It is preferable that the swallowing-in-progress frame includes an overexposure frame, a shielding frame, a flow frame, a colored water inflow frame, or a swallowing food frame. It is preferable that the post-swallowing frame includes a post-swallowing blur frame or a blackout frame.

It is preferable that, in a case in which the control processor classifies the examination image into a plurality of lightness information frames in accordance with lightness, the control processor determines, based on an expression pattern including the lightness information frame, the swallowing motion-specific frame, or the motion-specific swallowing block, whether the expression pattern indicates that the swallowing is in progress or that the swallowing is not in progress. It is preferable that the lightness information frame includes a high-lightness frame, a medium-lightness frame, or a low-lightness frame.

It is preferable that, in a case in which the control processor classifies a frame in which a blur of the examination image is equal to or less than a blur threshold value, as a pre-swallowing blur frame, the control processor determines, based on an expression pattern including the pre-swallowing blur frame, the swallowing motion-specific frame, or the motion-specific swallowing block, whether the expression pattern indicates that the swallowing is in progress or that the swallowing is not in progress. It is preferable that the control processor calculates, for each frame, a value of a weighting function for evaluating whether the swallowing is in progress or the swallowing is not in progress, and changes a type of the frame to the swallowing-in-progress frame or the swallowing-not-in-progress frame based on the value of the weighting function.

Still another aspect of the present invention relates to an operation method of an image processing device, the operation method comprising: causing a control processor to execute a step of acquiring an examination image, a step of classifying each frame of the examination image into any one of a swallowing-in-progress frame in which swallowing is in progress or a swallowing-not-in-progress frame in which the swallowing is not in progress, and a step of integrating, in a case in which a temporal interval between the swallowing-in-progress frames temporally adjacent to each other is equal to or less than a first threshold value, at least the adjacent swallowing-in-progress frames as a swallowing block.

It is preferable that the control processor determines that the swallowing-in-progress frame of which a temporal width is equal to or less than a second threshold value or equal to or more than a third threshold value is not the swallowing-in-progress frame, or determines that the swallowing block of which a temporal width is equal to or less than the second threshold value or equal to or more than the third threshold value is not the swallowing block.

According to the present invention, it is possible to prevent oversight during the examination or reduce the burden on the user in the video endoscopic examination of swallowing.

### BRIEF DESCRIPTION OF THE DRAWINGS

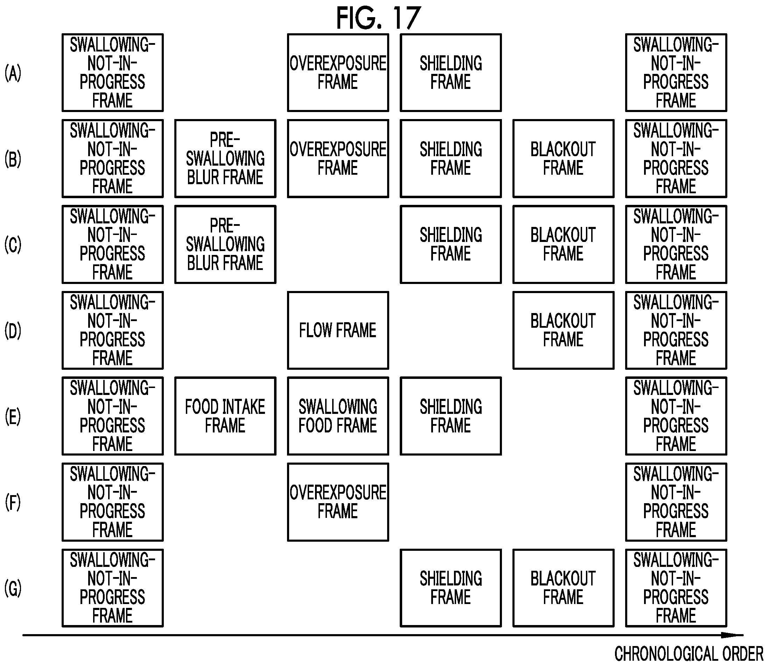

Fig. 1 is a schematic diagram of an endoscope system.
Fig. 2 is an explanatory diagram showing swallowing.
Fig. 3 is an explanatory diagram showing aspiration.
Fig. 4 is an explanatory diagram and an image diagram showing an imaging method of a video endoscopic examination of swallowing.
Fig. 5 is a block diagram showing a function of a swallowing determination unit in a first embodiment.
Fig. 6 is an explanatory diagram showing a swallowing-in-progress frame and a swallowing-not-in-progress frame.
Fig. 7 is an explanatory diagram showing integration of the swallowing-in-progress frames in the first embodiment.
Fig. 8 is an explanatory diagram showing a determination of a temporal width in the first embodiment.
Fig. 9 is a graph showing a histogram of a temporal interval of the swallowing-in-progress frame.
Fig. 10 is a graph showing the histogram of the temporal width of the swallowing-in-progress frame.
Fig. 11 is a graph showing the histogram of the temporal width of the swallowing-in-progress frame for which a user makes a diagnosis as being a correct answer and the histogram of the temporal width of the swallowing-in-progress frame for which the user makes a diagnosis as being an incorrect answer.
Fig. 12 is a flowchart showing the series of flows according to the embodiment of the present invention.
Fig. 13 is a block diagram showing a function of a swallowing determination unit in a second embodiment.
Fig. 14 is an explanatory diagram in which examination images during a period in which the swallowing is not in progress, immediately before the swallowing, during a period in which the swallowing is in progress, immediately after the swallowing, and during a period in which the swallowing is not in progress are arranged in a chronological order in association with swallowing motion.
Fig. 15 is explanatory diagram showing integration of shielding frames in the second embodiment.
Fig. 16 is explanatory diagram showing a determination of a temporal width in the second embodiment.
   (A) to (E) of Fig. 17 are explanatory diagrams showing expression patterns that do not correspond to an expression pattern during a period in which the swallowing is not in progress, and (F) and (G) of Fig. 17 are explanatory diagrams showing expression patterns that correspond to the expression pattern during a period in which the swallowing is not in progress.

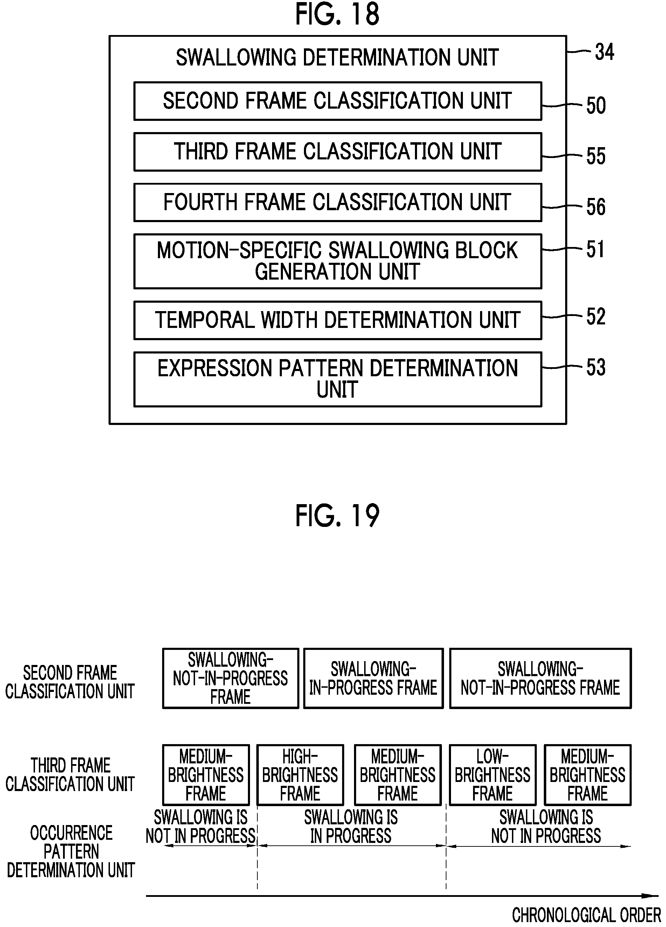

Fig. 18 is a block diagram showing a function of a swallowing determination unit in a third embodiment.
Fig. 19 is an explanatory diagram in a case in which an expression pattern determination unit determines that the swallowing is in progress based on classification in a second frame classification unit and a third frame classification unit.

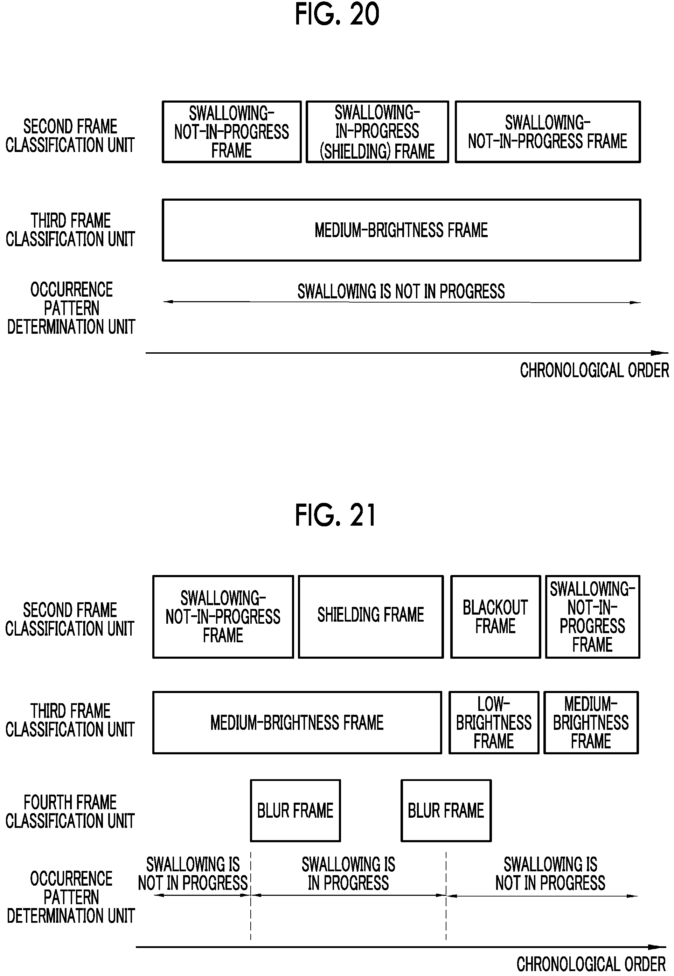

Fig. 20 is an explanatory diagram in a case in which an expression pattern determination unit determines that the swallowing is not in progress based on the classification in the second frame classification unit and the third frame classification unit.
Fig. 21 is an explanatory diagram in a case in which the expression pattern determination unit determines that the swallowing is in progress based on the classification in the second frame classification unit, the third frame classification unit, or a fourth frame classification unit.

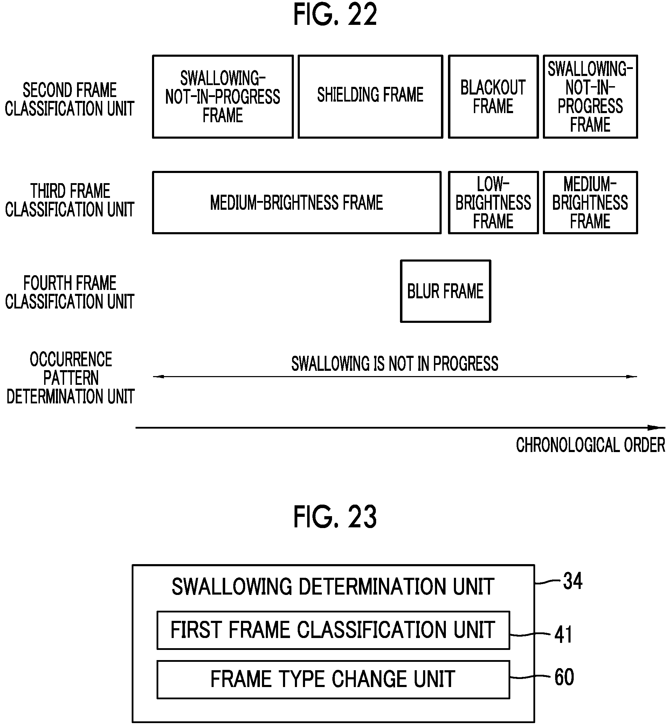

Fig. 22 is an explanatory diagram in a case in which the expression pattern determination unit determines that the swallowing is not in progress based on the classification in the second frame classification unit, the third frame classification unit, or the fourth frame classification unit.
Fig. 23 is a block diagram showing a function of a swallowing determination unit in a different case from the first to third embodiments.

### DESCRIPTION OF THE PREFERRED EMBODIMENTS

### First Embodiment

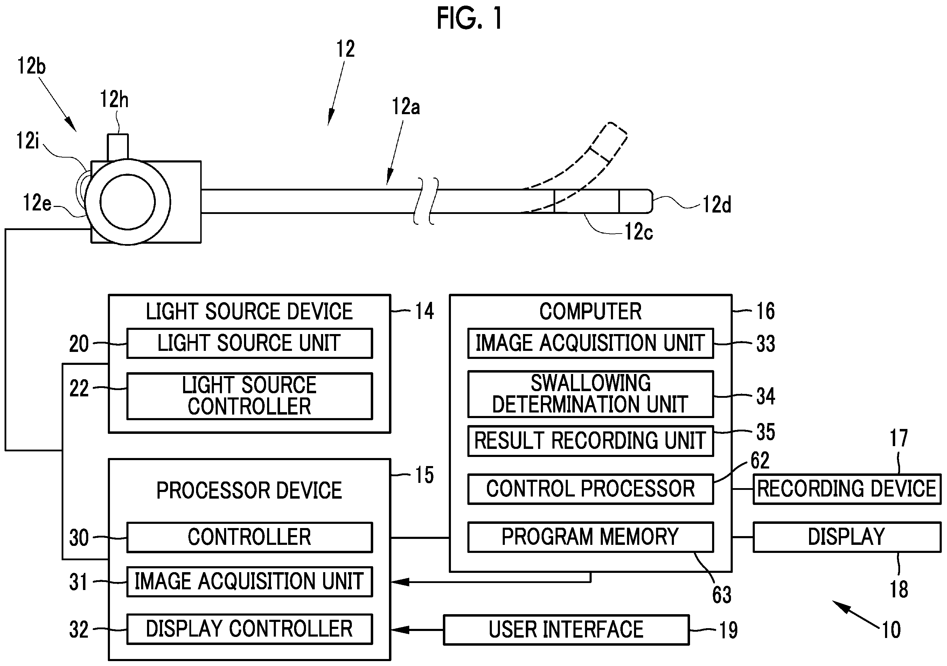

As shown in Fig. 1, an endoscope system 10 comprises an endoscope 12, a light source device 14, a processor device 15, a computer 16, a recording device 17, a display 18, and a user interface 19. The endoscope 12 is optically connected to the light source device 14, and is electrically connected to the processor device 15. The endoscope 12 includes an insertion part 12a to be inserted into a body of an observation target, an operating part 12b provided at a base end portion of the insertion part 12a, and a bendable part 12c and a distal end part 12d provided on a distal end side of the insertion part 12a. The bendable part 12c is operated in a bendable manner by operating an angle knob 12e of the operating part 12b. As the bendable part 12c is operated in a bendable manner, the distal end part 12d is made to face in a desired direction. The endoscope 12 is an endoscope used for a video endoscopic examination of swallowing.

An imaging optical system for forming a subject image and an illumination optical system for irradiating a subject with illumination light are provided in the endoscope 12. The subject is an in-vivo structure related to swallowing movement. Specifically, a pharyngeal part and a laryngeal part are included. The illumination light passes through the insertion part 12a of the endoscope 12 via a light guide and is emitted toward the subject from the distal end part 12d via an illumination lens of the illumination optical system. It should be noted that, in a case in which the light source unit 20 is built in the distal end part 12d of the endoscope, the light source unit 20 emits light to the subject via the illumination lens of the illumination optical system without passing through the light guide.

The imaging optical system is provided with an objective lens and an imaging sensor. The light from the observation target due to the irradiation with the illumination light is incident on the imaging sensor through the objective lens and a zoom lens. Therefore, an image of the observation target is formed on the imaging sensor. The zoom lens is a lens for magnifying the observation target, and is moved between a telephoto end and a wide end by operating a zoom operation unit 12i. The imaging sensor may be disposed in the distal end part 12d of the endoscope, or may be a so-called fiberscope that uses a fiber bundle in the insertion part of the endoscope 12 and located at the end of the insertion part 12a on the operating part side.

The imaging sensor is a complementary metal-oxide-semiconductor (CMOS) sensor, a charge-coupled device (CCD) sensor, or the like. An examination image is generated based on an image signal detected by the imaging sensor.

The imaging sensor may include a monochrome imaging sensor in which a color filter that converts sensed light into a monochrome image signal is not provided, in addition to a color imaging sensor in which a color filter (Bayer filter or the like) that converts the sensed light into a color image signal is provided. It should be noted that the color imaging sensor may convert the sensed light into a CMY signal, instead of an RBG signal.

In a case in which a color image is acquired, the image signal includes a B image signal output from a B pixel, a G image signal output from a G pixel, and an R image signal output from an R pixel. The image signal is output to the image acquisition unit 31 of the processor device 15 and is acquired as an examination image that is a monochrome image or the color image. The examination image acquired by the image acquisition unit 31 is output to an image acquisition unit 33 of the computer 16. The examination image output to the image acquisition unit 33 is output to a swallowing determination unit 34. The examination image is a still image captured during the endoscopic examination or the series of time-series videos captured during the endoscopic examination.

The operating part 12b is provided with a still image acquisition instruction switch 12h that is used to issue an instruction to acquire a still image of the observation target and a zoom operation unit 12i that is used to operate the zoom lens, in addition to the angle knob 12e.

The light source device 14 generates the illumination light. The processor device 15 performs system control of the endoscope system 10, image processing on the image signal output from the endoscope 12, and the like. The display 18 is a display unit that displays an image captured by the endoscope 12. The user interface 19 is an input device used to input settings and the like to the processor device 15 and the like.

The light source device 14 comprises a light source unit 20 that emits the illumination light, and a light source controller 22 that controls an operation of the light source unit 20. The light source unit 20 emits the illumination light for illuminating the subject. The light source unit 20 includes a light source, such as a laser diode, a light emitting diode (LED), a xenon lamp, or a halogen lamp. The light source controller 22 controls turning-on or turning-off of each light source constituting the light source unit 20, an amount of light emitted from each light source, and the like.

It should be noted that the light source unit 20 may be built in the endoscope 12. In addition, the light source controller 22 may be built in the endoscope 12 or may be built in the processor device 15. A white color includes so-called pseudo-white color which is obtained by mixing violet light V, blue light B, green light G, or red light R and which is substantially equivalent to white color in the imaging of the subject using the endoscope 12. A light source that emits ultraviolet light or infrared light for special light observation may be further included. Further, the light source unit 20 includes, as necessary, an optical filter or the like that adjusts a wavelength range, a spectrum, a light amount, or the like of the illumination light. For example, the light source unit 20 may perform high-speed irradiation (high-speed switching) by sequentially switching the blue light B, the green light G, and the red light R, the imaging sensor may acquire an image of each illumination light color by the monochrome sensor or the color sensor, and the processor may combine the images to generate a white image. In the light source unit 20, as a mechanism for switching the illumination light of a plurality of wavelengths at a high speed, there is a method of mechanically switching a plurality of color filters of different colors with respect to a white light source such as a xenon lamp, or a method of electronically switching ON/OFF of a plurality of LEDs that emit different colors.

The processor device 15 includes a controller 30, the image acquisition unit 31, and a display controller 32. In the processor device 15, a program in a program memory is operated by the controller 30 composed of a processor, to implement the functions of the image acquisition unit 31 and the display controller 32.

The computer 16 (image processing device) includes the image acquisition unit 33, the swallowing determination unit 34, a result recording unit 35, a control processor 62, and a program memory 63. In the computer 16, a program in the program memory 63 is operated by the control processor 62, to implement the functions of the image acquisition unit 33, the swallowing determination unit 34, and the result recording unit 35. It should be noted that the computer 16 and/or the light source controller 22 may be included in the processor device 15. The result recording unit 35 generates an image to be displayed on the display 18 and an image to be output to the recording device 17, and edits a video.

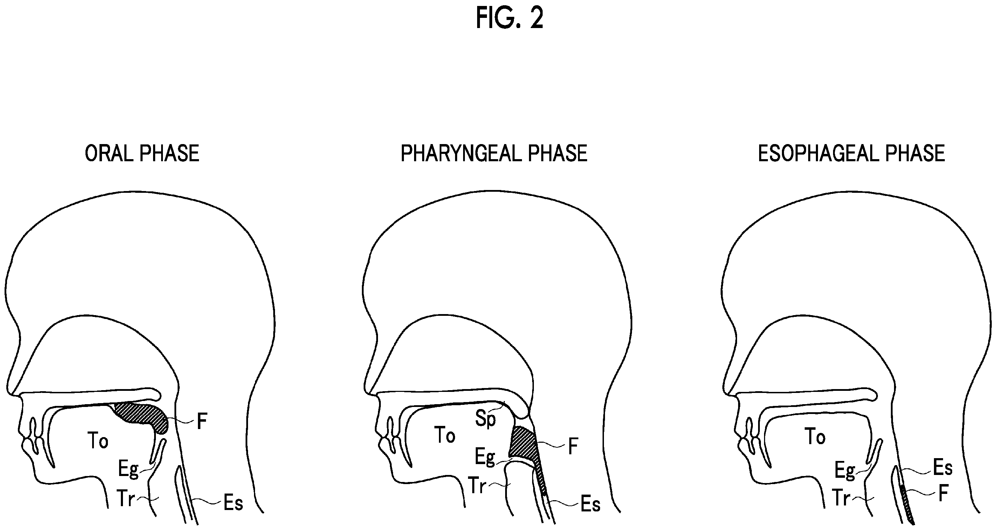

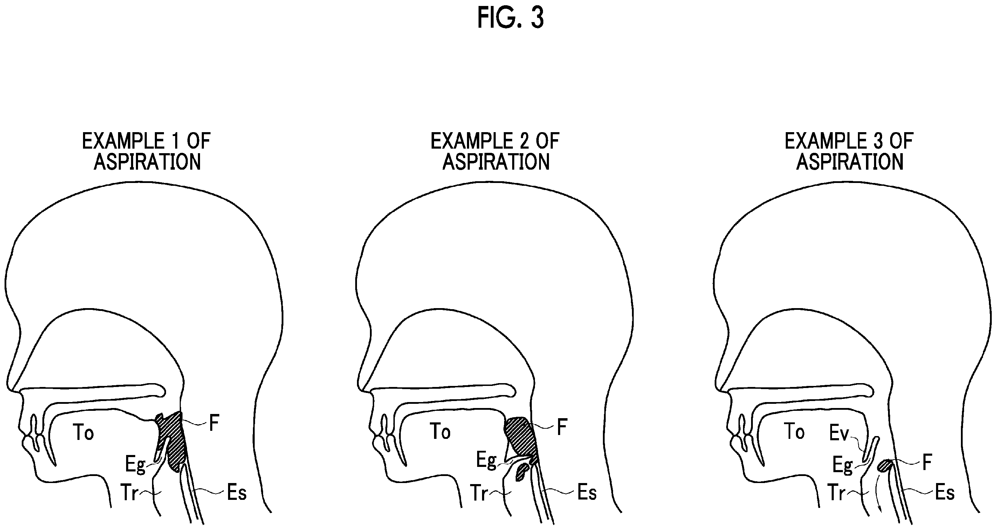

Hereinafter, the function of the swallowing determination unit 34 will be described. The swallowing determination unit 34 determines whether the swallowing is in progress or the swallowing is not in progress in the acquired examination image. The swallowing refers to the series of motions of putting food or drink into the mouth, chewing the food or drink, swallowing the food or drink, and sending the food or drink to the esophagus. Figs. 2 and 3 are explanatory diagrams of normal swallowing and abnormal swallowing (aspiration). As shown in Fig. 2, the swallowing movement is divided into an "oral phase" in which food F is carried from the oral cavity to the pharynx mainly by the movement of a tongue To, a "pharyngeal phase" in which the food F is carried from the pharynx to an esophagus Es by a swallowing reflex, and an "esophageal phase" in which the food F is carried from the esophagus Es to the stomach by peristaltic movement of the esophagus.

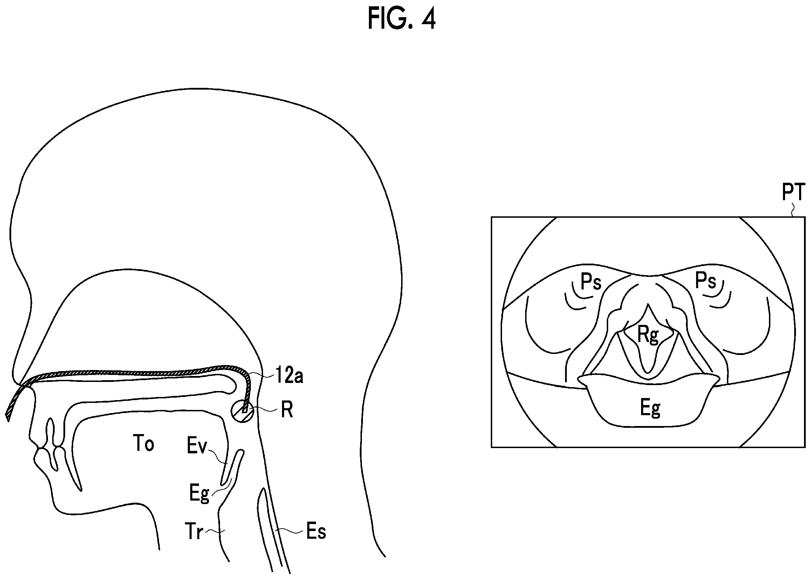

During the swallowing, in order to direct the food F toward the esophagus Es and to prevent the food F from flowing into the trachea Tr, the epiglottis Eg that plays a role of covering the trachea Tr closes the entrance (glottis) of the trachea Tr by a reflex movement. A soft palate Sp, which is a ceiling of the oral cavity, also moves backward to close a passage between the oral cavity and the nasal cavity, thereby preventing the food F from entering the nasal cavity. In a case in which any dysfunction occurs at any timing of the oral phase, the pharyngeal phase, or the esophageal phase, as shown in Fig. 4, the inflow of the food F, which is supposed to be carried to the esophagus Es in a normal case, into the trachea Tr is referred to as the aspiration.

Example 1 of the aspiration in Fig. 3 is an example of the aspiration in which the food F flows into the trachea Tr before the swallowing reflex occurs from the oral phase to the pharyngeal phase. Example 2 of the aspiration in Fig. 3 is an example of the aspiration in which the food F flows into the trachea Tr due to incomplete closure of the glottis (entrance of the trachea Tr) by the epiglottis Eg in the middle of the swallowing reflex from the pharyngeal phase to the esophageal phase. Example 3 of the aspiration in Fig. 3 is an example of the aspiration in which the food F remaining in an epiglottic vallecula Ev or a pyriform sinus (see an examination image PT in Fig. 4) which is a depression present on the left and right sides of the entrance of the esophagus flows into the trachea Tr after the swallowing reflex.

The examination image acquired in the present embodiment is acquired by inserting the insertion part 12a of the endoscope 12 from the nasal cavity to the pharynx and imaging the examination image such that the distal end part 12d of the endoscope is located near a position R of an oropharyngeal part shown in Fig. 4. It is preferable that the examination image includes anatomical structures such as the epiglottis Eg, a rima glottidis Rg, and right and left pyriform sinuses Ps as shown in the examination image PT of Fig. 4. The rima glottidis Rg is a space between the left and right folds constituting the vocal cords. Hereinafter, a case will be described in which the distal end part 12d of the endoscope is disposed in the oropharyngeal part, but the distal end part 12d may be disposed in the nasopharyngeal part, the nasopharyngeal part, the hypopharyngeal part, or the laryngeal part to perform the swallowing determination.

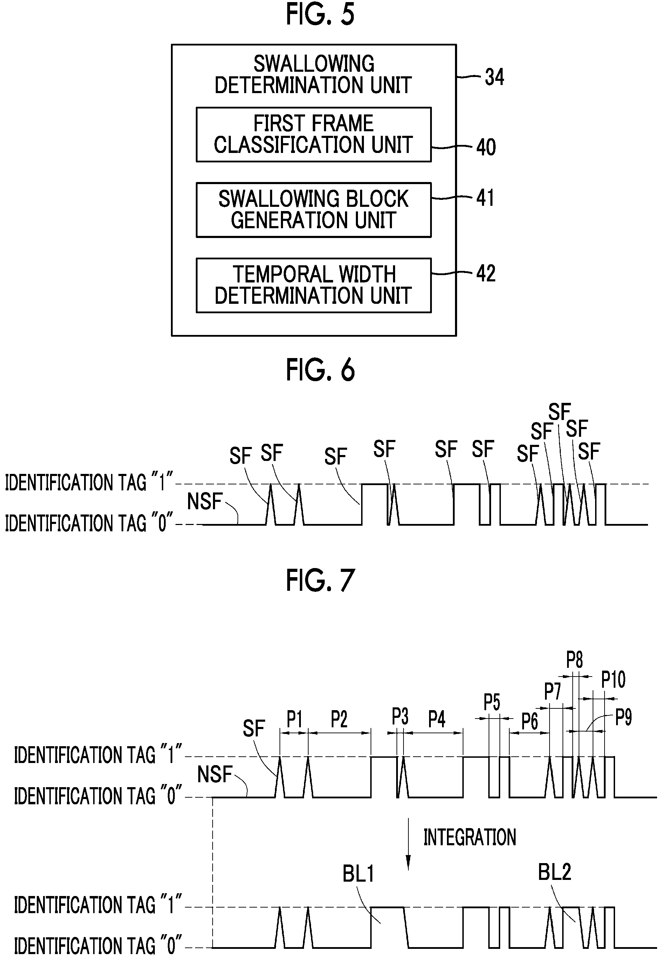

As shown in Fig. 5, the swallowing determination unit 34 comprises a first frame classification unit 40, a swallowing block generation unit 41, and a temporal width determination unit 42. The first frame classification unit 40 classifies each frame of the examination image into any one of a swallowing-in-progress frame in which the swallowing is in progress or a swallowing-not-in-progress frame in which the swallowing is not in progress. Specifically, in a case in which the first frame classification unit 40 classifies the examination image acquired in a frame unit as the swallowing-in-progress frame, an identification tag " 1" indicating the swallowing-in-progress frame is assigned to the examination image. On the other hand, in a case in which the first frame classification unit 40 classifies the examination image as the swallowing-not-in-progress frame, an identification tag "0" indicating the swallowing-not-in-progress frame is assigned to the examination image. In a case in which these identification tags " 1" and "0" are represented in time series, for example, as shown in Fig. 6. The identification tag " 1" indicates that the examination image to which the identification tag " 1" is assigned is a swallowing-in-progress frame SF, and the identification tag "0" indicates that the examination image to which the identification tag "0" is assigned is a swallowing-not-in-progress frame NSF.

It should be noted that it is preferable that the first frame classification unit 40 is a learning model trained through deep learning (the same applies to a second frame classification unit 50 described below). Specifically, it is preferable that the first frame classification unit 40 learns the examination image of the swallowing-in-progress frame and the examination image of the swallowing-not-in-progress frame, in advance through machine learning. The machine learning may use unsupervised learning or semi-supervised learning that automatically clusters the images of the swallowing-in-progress frame or the swallowing-not-in-progress frame. It is known that one swallowing usually takes a time of about several hundreds of milliseconds to 2 seconds. In a swallowing detection method in the related art, images in which it is correctly determined that the swallowing is in progress and images in which it is determined that the swallowing is not in progress are mixed, and thus it appears that there are a plurality of times of swallowing.

The deep learning is known as a method excellent in image recognition, and is an excellent method of extracting features from one image, detecting a target pattern, and performing segmentation or classification. However, it is known that a field called motion recognition, which recognizes what kind of motion is taken between images arranged in a chronological order, is technically more difficult than simple image recognition. In the present invention, the swallowing block generation unit 41 and temporal width determination unit 42 are used for processing of complementing the motion recognition during a period in which the swallowing is in progress over a plurality of frames.

In a case in which a temporal interval between the swallowing-in-progress frames temporally adjacent to each other is equal to or less than a first threshold value, the swallowing block generation unit 41 integrates at least the adjacent swallowing-in-progress frames as a swallowing block. Specifically, as shown in Fig. 7, the swallowing block generation unit 41 calculates temporal intervals P1 to P10 between the adjacent swallowing-in-progress frames. Adjacent swallowing-in-progress frames CF1 and CF2 having the temporal intervals P3 and P8, which are equal to or less than the first threshold value among the temporal intervals P1 to P10, are integrated as swallowing blocks BL1 and BL2.

It should be noted that, as a method of integration, for example, it is preferable to perform processing of changing all the swallowing-not-in-progress frames between the adjacent swallowing-in-progress frames CF1 to the swallowing-in-progress frame.

It should be noted that it is preferable that the first threshold value is 0.6 seconds or the number of frames corresponding to 0.6 seconds. The number of frames corresponding to 0.6 seconds is, for example, 18 frames in a case in which a frame rate of the video is 30 frames (flame per second), and is, for example, 36 frames in a case in which the frame rate of the video is 60 frames. The reason why the first threshold value is 0.6 seconds will be described below. The temporal interval may be an interval represented by other units such as the number of frames determined from the frame rate and the number of pixels converted in the computer for executing the image processing, in addition to a temporal interval represented by seconds or the like.

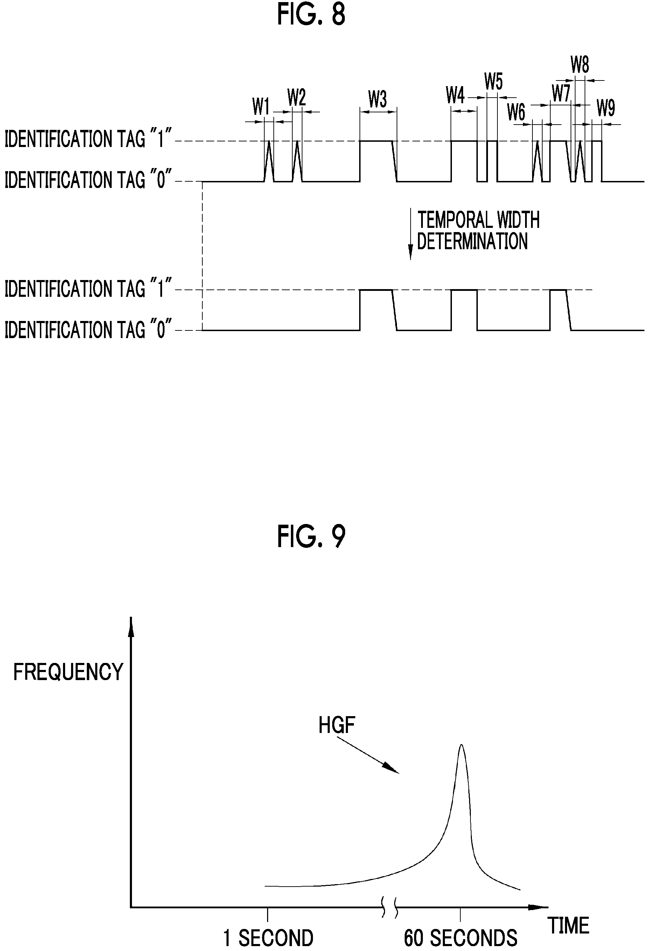

The temporal width determination unit 42 determines that the swallowing block of which the temporal width is equal to or less than a second threshold value or equal to or more than a third threshold value is not the swallowing block, or that the swallowing-in-progress frame of which the temporal width is equal to or less than the second threshold value or equal to or more than the third threshold value is not the swallowing-in-progress frame. Specifically, as shown in Fig. 8, the temporal width determination unit 42 calculates temporal widths W3 and W7 of the swallowing blocks, and calculates temporal widths W1, W2, and W3 to W9 of the swallowing-in-progress frames. Since the temporal widths W3 and W7 of the swallowing blocks BL1 and BL2 are not equal to or less than the second threshold value, the swallowing blocks BL1 and BL2 are determined as the swallowing blocks as they are. In accordance with the determination, the identification tag "1" is assigned to the swallowing blocks BL1 and BL2. The swallowing-in-progress frames having the temporal widths W1, W2, W5, W6, W8, and W9, which are equal to or less than the second threshold value among the temporal widths W1, W2, and W3 to W9, are determined as not being the swallowing-in-progress frames. In accordance with the determination, the identification tag of the swallowing-in-progress frames having the temporal widths W1, W2, W5, W6, W8, and W9 is changed from "1" to "0". On the other hand, the swallowing-in-progress frame having the temporal width W4 exceeding the second threshold value is determined as the swallowing-in-progress frame. Therefore, the identification tag "1" is maintained as it is in the swallowing-in-progress frame having the temporal width W4.

Further, the temporal width determination unit 42 determines that the swallowing block of which the temporal width is equal to or more than the third threshold value is not the swallowing block, or that the swallowing-in-progress frame of which the temporal width is equal to or more than the third threshold value is not the swallowing-in-progress frame. It is preferable that the third threshold value is 1.8 seconds or 54 frames which is the number of frames corresponding to 1.8 seconds (in a case in which the frame rate of the video is 30 frames (flame per second)).

It should be noted that it is preferable that the second threshold value is 0.2 seconds or 6 frames which is the number of frames corresponding to 0.2 seconds (in a case in which the frame rate is 30 frames per second (fps)). The reason why the second threshold value is 0.2 seconds will be described below. In addition, the temporal width may be a width represented by other units such as a frame determined from the frame rate, in addition to the temporal width represented by a time such as seconds.

As described above, by assigning the identification tag "1" to the swallowing block or the swallowing-in-progress frame through the processing in the swallowing determination unit 34, the user can easily search for the examination image in which the swallowing is in progress, during the reproduction of the video during the diagnosis or after the diagnosis.

It should be noted that the reason why the first threshold value is set to 0.6 seconds is as follows. Fig. 9 shows a histogram HGF of the temporal interval of the swallowing-in-progress frame for which the user who is actually familiar with the video endoscopic examination of swallowing diagnoses that the swallowing is in progress from the swallowing endoscope video or the swallowing-in-progress frame for which the user diagnoses that the swallowing is not in progress. In the histogram HGF, a median value is 8 seconds, a minimum value is 0.5 seconds, and a maximum value is 60 seconds. In addition, there are almost no swallowing-in-progress frames of which the temporal interval is equal to or less than 0.5 seconds. From the above-described consideration on the histogram HGF, it is preferable that the first threshold value indicating a boundary value for determining whether or not to be integrated into the swallowing block is set to 0.6 seconds.

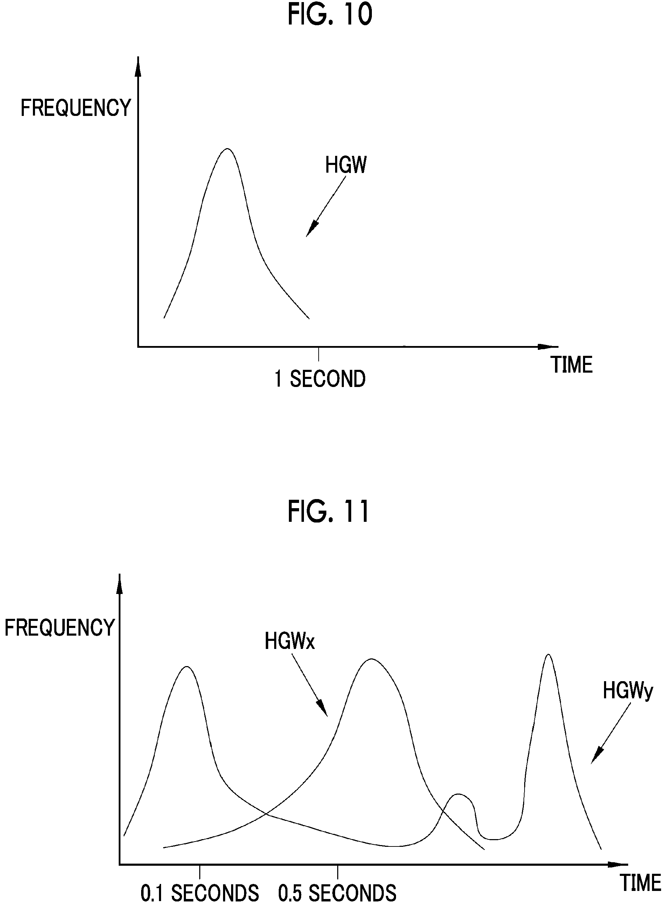

It should be noted that the reason why the second threshold value is set to 0.2 seconds is as follows. Fig. 10 shows a histogram HGW of the temporal width of the swallowing-in-progress frame for which the user who is actually familiar with the video endoscopic examination of swallowing diagnoses that the swallowing is in progress from the swallowing endoscope video or the swallowing-in-progress frame for which the user diagnoses that the swallowing is not in progress. In the histogram HGW, a median value is 0.6 seconds, a minimum value is 0.1 seconds, and a maximum value is 0.9 seconds. In addition, Fig. 11 shows a histogram HGWx of the temporal width of the swallowing-in-progress frame (true positive (TP)) that is correctly classified as compared to the user's diagnosis among the swallowing-in-progress frames classified by the first frame classification unit 40 and a histogram HGWy of the temporal width of the swallowing-in-progress frame (false positive (FP)) that is incorrectly classified. The histogram HGWx and the histogram HGWy are separated from each other. In the histogram HGWx, the frequency equal to or less than 0.1 seconds (3 frames) is high. In the histogram HGWy, the frequency equal to or more than 0.5 seconds (15 frames) is low. From the above-described consideration on the histograms HGW, HGWx, and HGWy, it is preferable that the second threshold value indicating a boundary value for determining whether the swallowing is in progress or the swallowing is not in progress is set to 0.2 seconds in a range from 0.1 to 0.5 seconds.

The reason why the third threshold value is set to 1.8 seconds is as follows. As shown in the histogram HGW, even in a case in which a low occurrence probability is considered, the swallowing-in-progress frame having a temporal width that is twice the maximum value, that is, a temporal width exceeding 1.8 seconds can be regarded as an erroneous classification. Therefore, the third threshold value is preferably 1.8 seconds.

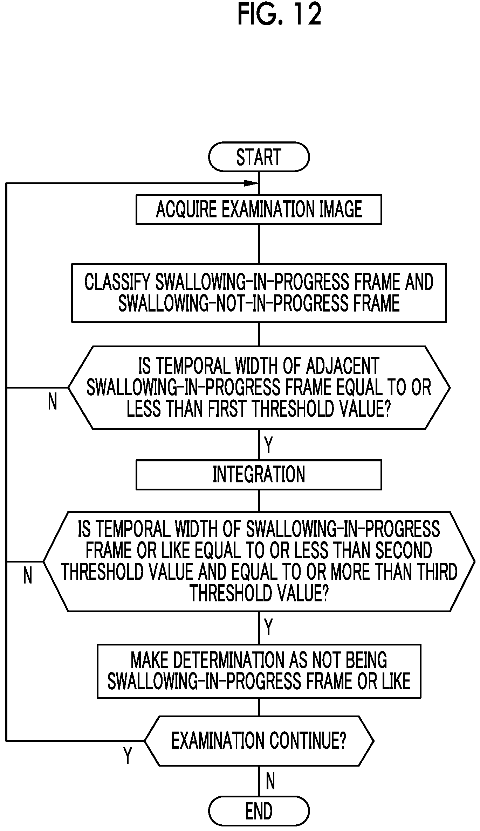

Next, the series of flows according to the embodiment of the present invention will be described with reference to a flowchart of Fig. 12. The image acquisition unit 33 acquires the examination image. The first frame classification unit 40 classifies each frame of the examination image into any one of the swallowing-in-progress frame in which the swallowing is in progress or the swallowing-not-in-progress frame in which the swallowing is not in progress. In a case in which the temporal interval between the swallowing-in-progress frames temporally adjacent to each other is equal to or less than a first threshold value, the swallowing block generation unit 41 integrates at least the adjacent swallowing-in-progress frames as a swallowing block. For example, the adjacent swallowing-in-progress frames CF1 and CF2 having the temporal intervals P3 and P8 which are equal to or less than the first threshold value are integrated as the swallowing blocks BL1 and BL2 (see Fig. 7).

The temporal width determination unit 42 determines that the swallowing-in-progress frame of which the temporal width is equal to or less than the second threshold value or equal to or more than the third threshold value is not the swallowing-in-progress frame, or determines that the swallowing block of which the temporal width is equal to or less than the second threshold value or equal to or more than the third threshold value is not the swallowing block. The identification tag " 1" indicating that the swallowing is in progress is assigned to the swallowing block and the swallowing-in-progress frame. The above-described series of processing is repeatedly performed as long as the swallowing examination is pending.

### Second Embodiment

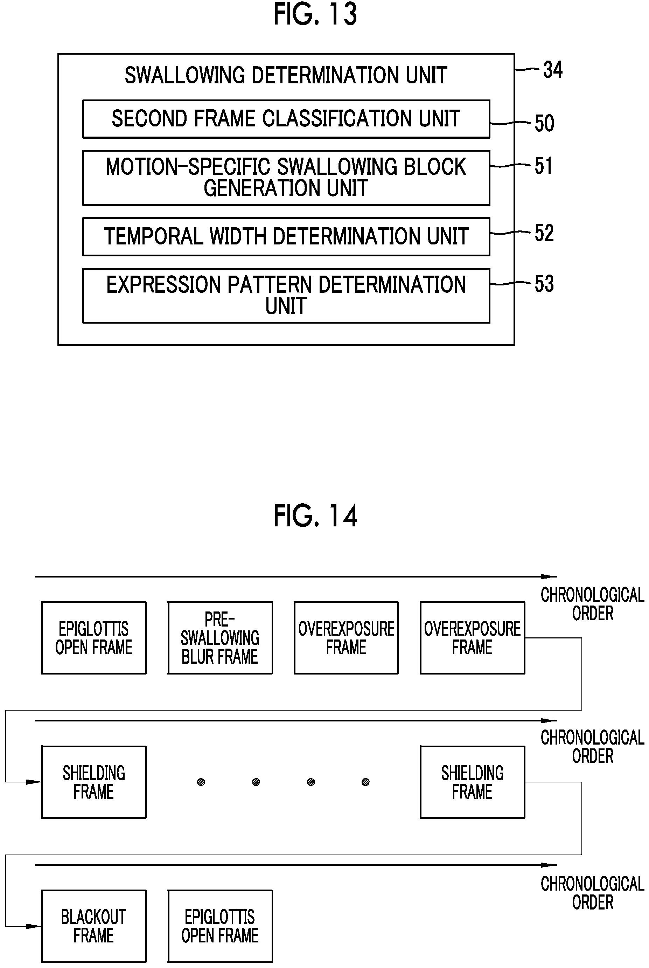

In the second embodiment, the swallowing determination unit 34 classifies the examination images in accordance with the motion during a period in which the swallowing is in progress. As shown in Fig. 13, the swallowing determination unit 34 comprises the second frame classification unit 50, a motion-specific swallowing block generation unit 51, a temporal width determination unit 52, and an expression pattern determination unit 53. It should be noted that, since the other units except for the swallowing determination unit 34 are the same as those in the first embodiment, the description thereof will be omitted.

The second frame classification unit 50 classifies each frame of the examination image into any of a plurality of swallowing motion-specific frames in accordance with the swallowing motion or the swallowing-not-in-progress frame in which the swallowing is not in progress. Specifically, in a case in which the second frame classification unit 50 classifies the examination image acquired in a frame unit as the swallowing motion-specific frame, the examination image is classified as any one of the pre-swallowing frame, the swallowing-in-progress frame, or the post-swallowing frame as the swallowing motion-specific frame.

The swallowing-not-in-progress frame, the pre-swallowing frame, the swallowing-in-progress frame, or the post-swallowing frame is obtained as follows in accordance with the swallowing motion. Fig. 14 is an explanatory diagram in which the examination images during a period in which the swallowing is not in progress, immediately before the swallowing, during a period in which the swallowing is in progress, immediately after the swallowing, and during a period in which the swallowing is not in progress are arranged in a chronological order in association with the swallowing motion. During a period in which the swallowing is not in progress, an epiglottis open frame in which the epiglottis is open is obtained as the swallowing-not-in-progress frame. The epiglottis open frame is about 1 frame.

Immediately before the swallowing, as the pre-swallowing frame, a pre-swallowing blur frame is obtained in which the epiglottis moves at a high speed and a blur occurs. The pre-swallowing blur frame is about 1 frame. The blur of the pre-swallowing blur frame is characteristically represented by a movement such as lifting of the epiglottis or approach of a structure. It should be noted that, as the other pre-swallowing frame, an epiglottis inversion frame or a food intake frame is obtained.

During a period in which the swallowing is in progress, an overexposure frame in which a wide portion of the screen is covered with a white halation or a shielding frame in which the entire screen is out of focus and covered with a slightly dark tone is obtained as the swallowing-in-progress frame. The shielding frame appears after the overexposure frame. The overexposure frame is about 2 frames more than the pre-swallowing blur frame. The number of shielding frames is about 7 frames, which is more than the number of overexposure frames. It should be noted that other swallowing-in-progress frames include a flow frame in a process of swallowing the colored swallowing water, a colored water inflow frame, or a swallowing food frame. The colored water includes milk or a liquid such as green, yellow, or red obtained by mixing a food coloring agent with water. In addition, the colored water inflow frame may be classified into a plurality of coloring-specific frames in accordance with the type of the colored water. Similarly, the swallowing food frame may be classified into a plurality of swallowing food-specific frames in accordance with the type of the swallowing food. The swallowing food includes, for example, a fluid food such as pudding and a solid food such as rice and meat.

Immediately after the swallowing, a blackout frame in which the entire screen is dark is obtained as the post-swallowing frame. The blackout frame is about 1 frame. It should be noted that, as the other post-swallowing frames, a post-swallowing blur frame that is characteristically represented by the movement of the epiglottis is obtained.

It should be noted that, in a case in which the second frame classification unit 50 classifies the examination image as the swallowing motion-specific frame, the identification tag "1" indicating that the swallowing motion is being performed is assigned to the examination image. On the other hand, in a case in which the examination image is classified as the swallowing-not-in-progress frame, the identification tag "0" indicating the swallowing-not-in-progress frame is assigned to the examination image.

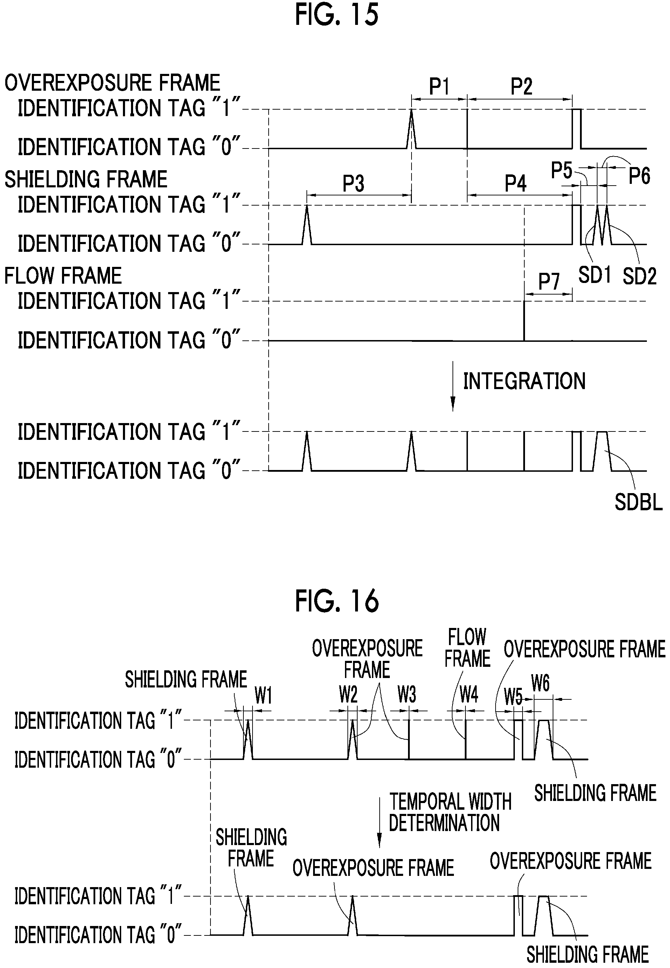

In a case in which a temporal interval between the swallowing motion-specific frames temporally adjacent to each other is equal to or less than the first threshold value, the motion-specific swallowing block generation unit 51 integrates at least the adjacent swallowing motion-specific frames as a motion-specific swallowing block. Specifically, as shown in Fig. 15, in a case in which the second frame classification unit 50 performs the classification into the overexposure frame, the shielding frame, and the flow frame, the motion-specific swallowing block generation unit 51 calculates the temporal intervals P1 to P7 between the adjacent swallowing motion-specific frames. Adjacent shielding frames SD1 and SD2 having the temporal interval P6, which is equal to or less than the first threshold value among the temporal intervals P1 to P7, are integrated as a shielding block SDBL as the swallowing motion-specific frame. It should be noted that it is preferable that the first threshold value is 1 second or a time corresponding to 30 frames (in a case in which the frame rate is 30 frames per second (fps)), as in the first embodiment.

The temporal width determination unit 52 determines that a swallowing motion-specific block of which the temporal width is equal to or less than the second threshold value or equal to or more than the third threshold value is not the swallowing motion-specific block, or that the swallowing motion-specific frame of which the temporal width is equal to or less than the second threshold value or equal to or more than the third threshold value is not the swallowing motion-specific frame. Specifically, as shown in Fig. 16, the temporal width determination unit 52 calculates the temporal width W6 of the shielding block SDBL, which is the swallowing motion-specific block, and calculates the temporal widths W1 to W5 of the shielding frame, the overexposure frame, and the flow frame. Since the temporal width W6 of the shielding block SDBL is not equal to or less than the second threshold value, the shielding block SDBL is determined as the swallowing block as it is. In accordance with the determination, the identification tag "1" is assigned to the shielding block SDBL. It should be noted that it is preferable that the second threshold value is 0.2 second or a time corresponding to 30 frames (in a case in which the frame rate is 6 frames per second (fps)), as in the first embodiment.

The shielding frames having the temporal width W1 exceeding the second threshold value or the overexposure frame having the temporal widths W2 and W5 exceeding the second threshold value are determined as the swallowing-in-progress frame. In this case, the identification tag " 1" is maintained as it is. On the other hand, the overexposure frame having the temporal width W3 equal to or less than the second threshold value or the flow frame having the temporal width W4 equal to or less than the second threshold value is determined as not being the swallowing-in-progress frame. In accordance with the determination, the identification tag is changed from "1" to "0".

The expression pattern determination unit 53 determines, based on an expression pattern including the swallowing motion-specific frame or the motion-specific swallowing block, whether the expression pattern indicates that the swallowing is in progress or that the swallowing is not in progress. The expression pattern represents a pattern of classification in time series. Specifically, since none of the expression patterns of (A) to (E) of Fig. 17 correspond to a swallowing-not-in-progress expression pattern, these expression patterns are determined as being during a period in which the swallowing is in progress.

(A) of Fig. 17 is an expression pattern that occurs in an order of the swallowing-not-in-progress frame, the overexposure frame, the shielding frame, and the swallowing-not-in-progress frame. (B) of Fig. 17 is an expression pattern that occurs in an order of the swallowing-not-in-progress frame, the pre-swallowing blur frame, the overexposure frame, the shielding frame, the blackout frame, and the swallowing-not-in-progress frame. (C) of Fig. 17 is an expression pattern that occurs in an order of the swallowing-not-in-progress frame, the pre-swallowing blur frame, the shielding frame, the blackout frame, and the swallowing-not-in-progress frame. (D) of Fig. 17 is an expression pattern that occurs in an order of the swallowing-not-in-progress frame, the flow frame, the blackout frame, and the swallowing-not-in-progress frame. (E) of Fig. 17 is an expression pattern that occurs in an order of the swallowing-not-in-progress frame, the food intake frame, the swallowing food frame, the shielding frame, and the swallowing-not-in-progress frame.

It should be noted that, as shown in (D) and (E) of Fig. 17, there is also a swallowing motion in which the overexposure frame is not generated. Accordingly, it is possible to accurately perform the swallowing determination even in a case in which a high pixel value region does not appear in a patient with a weakened swallowing function. In addition, as shown in (D) of Fig. 17, in a case in which the colored swallowing water is swallowed, it can be determined that the swallowing is in progress by the determination of the flow frame in a case in which there is no pre-swallowing blur frame or overexposure frame. In addition, as shown in (E) of Fig. 17, in a case in which the swallowing food is swallowed, the characteristic pattern of the swallowing food can also be a point that determines the swallowing.

On the other hand, since both the expression patterns in (F) and (G) of Fig. 17 correspond to the swallowing-not-in-progress expression pattern, these expression patterns are determined as being during a period in which the swallowing is not in progress. (F) of Fig. 17 is an expression pattern that occurs in an order of the swallowing-not-in-progress frame, the overexposure frame, and the swallowing-not-in-progress frame. In this case, the overexposure frame is generated because the distal end part 12d of the endoscope is close to a mucous membrane side wall, but the pre-swallowing blur frame or the shielding frame is not generated, so that it can be determined that the series of motions are during a period in which the swallowing is not in progress. (G) of Fig. 17 is an expression pattern that occurs in an order of the swallowing-not-in-progress frame, the shielding frame, the blackout frame, and the swallowing-not-in-progress frame. In this case, since the pre-swallowing blur frame and the overexposure frame are not present due to the adhesion of dirt on an imaging surface of the distal end part 12d of the endoscope, it is possible to determine that the series of motions are during a period in which the swallowing is not in progress.

### Third Embodiment

In the third embodiment, the swallowing determination unit 34 classifies the examination image in accordance with lightness and classifies the examination image in accordance with a blur, in addition to classifying the examination image in accordance with the motion during a period in which the swallowing is in progress as in the second embodiment. As shown in Fig. 18, the swallowing determination unit 34 comprises the second frame classification unit 50, a third frame classification unit 55, a fourth frame classification unit 56, the motion-specific swallowing block generation unit 51, the temporal width determination unit 52, and the expression pattern determination unit 53. It should be noted that, since the second frame classification unit 50, the motion-specific swallowing block generation unit 51, and the temporal width determination unit 52 are the same as those in the second embodiment, the description thereof will be omitted.

The third frame classification unit 55 classifies the examination images into a plurality of lightness information frames in accordance with the lightness. The lightness information frame includes a high-lightness frame, a medium-lightness frame, or a low-lightness frame in accordance with a magnitude of the lightness. The magnitude of the lightness of a certain frame is, for example, an average value (minimum 0, maximum 255) of the lightness in the HLS color space for all pixels within the frame, and the lightness equal to or more than 130 is defined as high lightness, the lightness equal to or more than 50 and less than 130 is defined as medium lightness, and the lightness less than 50 is defined as low lightness. More desirably, a method may be used in which an average value and a standard deviation of the lightness per frame in a certain period of time, for example, 10 seconds are calculated, a high-lightness threshold value and a medium-lightness threshold value are set as (average value + standard deviation), a medium-lightness threshold value and a low-lightness threshold value are set as (average value - standard deviation), and the threshold values are sequentially calculated by using a movement average method.

The expression pattern determination unit 53 determines whether the expression pattern indicates that the swallowing is in progress or that the swallowing is not in progress based on the expression pattern including the lightness information frame, the swallowing motion-specific frame, or the motion-specific swallowing block. Specifically, as shown in Fig. 19, in a case of the expression pattern in which the high-lightness frame is present before the swallowing-in-progress frame or overlaps with the swallowing-in-progress frame, and the low-lightness frame is generated after the swallowing-in-progress frame, the expression pattern determination unit 53 determines that the frame from the high-lightness frame to the end of the swallowing-in-progress frame is during a period in which the swallowing is in progress. It should be noted that it is preferable that the expression pattern determination unit 53 determines that the frame is during a period in which the swallowing is in progress even in a case in which the swallowing-in-progress frames are consecutively present within 0.6 seconds after the high-lightness frame in time.

On the other hand, as shown in Fig. 20, in a case in which the second frame classification unit 50 determines that the frame is the swallowing-in-progress frame, but the expression pattern is such that the medium-lightness frame continues in the third frame classification unit 55 before and after the swallowing-in-progress frame, the expression pattern determination unit 53 determines that the swallowing is not in progress. In addition, as another example, as a result of the third frame classification unit 55, even in a case in which the high-lightness frame, the medium-lightness frame, and the low-lightness frame appear in this order, in the second frame classification unit 50, it is determined that the swallowing is not in progress in a case in which there is no swallowing-in-progress frame. It should be noted that it is preferable that the expression pattern determination unit 53 determines that the swallowing is not in progress in a case in which the high-lightness frame is present alone or in a case in which the high-lightness frame is not present and the swallowing food frame is present alone. It is preferable that the expression pattern determination unit 53 determines that the swallowing is not in progress after the low-lightness frame appears in a case in which the low-lightness frame appears during the appearance of the swallowing-in-progress frame.

It should be noted that, in a case in which the examination image is classified in accordance with the lightness as in the third frame classification unit 55, the temporal width may be calculated in accordance with an appearance pattern of the pixel values of the image. In addition, the third frame classification unit 55 may be separated from the high-lightness frame in a case in which an area of a halation pixel of which the pixel value is more than a certain value is equal to or more than a certain area.

The fourth frame classification unit 56 classifies a frame in which the blur of the examination image is equal to or more than a blur threshold value as a blur frame. The expression pattern determination unit 53 determines whether the expression pattern indicates that the swallowing is in progress or that the swallowing is not in progress based on the expression pattern including the blur frame, the swallowing motion-specific frame, or the motion-specific swallowing block. In addition, the fourth frame classification unit 56 may perform the classification of the blur frame along or detect, in addition to or instead of the blur frame, any one of a blur amount, the magnitude of the image difference, a movement amount of the feature point, and then may determine whether the expression pattern based on the swallowing motion indicates that the swallowing is in progress or that the swallowing is not in progress by the expression pattern determination unit 53 based on the classification or the detection result and the frame classified by at least any one of the first frame classification unit 40, the second frame classification unit 50, the third frame classification unit 55, or the fourth frame classification unit 56.

For example, as shown in Fig. 21, in a case in which the fourth frame classification unit 56 classifies the frame immediately before the shielding frame as the blur frame and classifies the frame immediately before the low-lightness frame as the blur frame, the expression pattern determination unit 53 determines the blur frame immediately before the shielding frame as the first frame during a period in which the swallowing is in progress and determines the blur frame immediately before the low-lightness frame as the last frame during a period in which the swallowing is in progress. On the other hand, as shown in Fig. 22, in a case of an expression pattern in which there is no blur frame immediately before the shielding frame and the shielding frame and the blackout frame occur in this order, the expression pattern determination unit 53 determines that the swallowing is not in progress.

It should be noted that the best embodiment is as follows. The video obtained by the endoscope 12 is processed by the processor one frame at time. In the second frame classification unit 50, the image is classified into "swallowing-not-in-progress", "overexposure", "shielding", "colored water inflow", and "swallowing food". The second frame classification unit 50 is a classifier that has learned the images of the above-described classification in advance through the machine learning. Among these, "overexposure", "shielding", "colored water inflow", and "swallowing food" are all handled as the swallowing-in-progress frames, and "swallowing-not-in-progress" is handled as the swallowing-not-in-progress frame. In the swallowing block generation unit 41, the first threshold value is set to 0.6 seconds, and the integration into the swallowing block is performed.

At the same time, in the third frame classification unit 55, first, the average value of the brightness in the HLS color space is calculated as the lightness for all the pixels within the frame. In order to classify a certain frame, the average and standard deviation of the lightness in the previous 30 seconds are calculated, and the frame is classified as "high-lightness" in a case in which the lightness of the frame is equal to or more than the average + standard deviation, is classified as "low-lightness" in a case in which the lightness of the frame is less than the average - standard deviation, and is classified as "medium-lightness" in a case in which the lightness of the frame is between these values.

The expression pattern determination unit 53 determines whether the swallowing is in progress or the swallowing is not in progress in accordance with the expression pattern in the second frame classification unit 50, the third frame classification unit 55, or the fourth frame classification unit 56. For example, it is determined that the "swallowing is in progress" in a case in which there is the "high-lightness" frame and the "swallowing-in-progress frame" (swallowing block) follows within 0.6 seconds after the "high-lightness" frame in time, but it is determined that the "swallowing is not in progress" in a case in which the "high-lightness" frame is present alone or in a case in which the "high-lightness" frame is not present and the "swallowing food" frame is present alone. In addition, the swallowing-in-progress frame having the temporal width equal to or less than 0.2 seconds or equal to or more than 1.8 seconds is determined that the swallowing is not in progress. It should be noted that the pattern determined by the expression pattern determination unit 53 is not limited thereto, and a plurality of swallowing patterns and non-swallowing patterns are predetermined, and a determination is performed.

It should be noted that, in the above-described embodiments, the swallowing block generation unit 41 and the temporal width determination unit 42 are used to determine the swallowing-in-progress frame or the swallowing block, but the determination of the swallowing-in-progress frame or the like may be performed by other methods. For example, the change to the swallowing-in-progress frame or the frame may be performed by using the weighting function in accordance with the appearance frequency of the identification tags "0" and "1" between the adjacent swallowing-in-progress frames, corresponding to the time axis. In this case, as shown in Fig. 23, the swallowing determination unit 34 includes the swallowing block generation unit 41 and a frame type change unit 60 instead of the temporal width determination unit 42.

The frame type change unit 60 calculates a value of the weighting function for evaluating whether the swallowing is in progress or the swallowing is not in progress for each frame, and changes the type of the frame to the swallowing-in-progress frame or the swallowing-not-in-progress frame based on the value of the weighting function. The weighting function is a function for evaluating whether the swallowing is in progress or the swallowing is not in progress in the specific frame in accordance with the appearance frequency or the appearance pattern of the swallowing-in-progress frame and the swallowing-not-in-progress frame in the previous and subsequent time for the specific frame.

Specifically, in a case in which the swallowing-in-progress frame appears in the n-th frame, the weighting function of the frame is calculated as f(n) = 1. In a case in which the swallowing-not-in-progress frame appears in the next (n+1)-th frame, f(n+1) = f(n) - k is calculated by using a coefficient k (for example, k = 0.1). A function is set such that the value of the weighting function is decreased as the swallowing-not-in-progress frame is consecutive by setting f(n+2) = f(n+1) - k × 2 in a case in which the two swallowing-not-in-progress frames appear consecutively in the next (n+2)-th frame, and setting f(n+i) = f(n+i-1) - k × i in a case in which the swallowing-not-in-progress frame is consecutive up to the next (n+i)-th frame. In a case in which the value of the weighting function is equal to or more than the type change threshold value (for example, 0.5), the frame type change unit 60 maintains the swallowing-in-progress frame as it is, and changes the swallowing-not-in-progress frame to the swallowing-in-progress frame. On the other hand, in a case in which the value of the weighting function is less than the type change threshold value, the frame type change unit 60 maintains the swallowing-not-in-progress frame as it is, and changes the swallowing-in-progress frame to the swallowing-not-in-progress frame.

In addition, as another form of the weighting function, the identification tags "0" and " 1" between the adjacent swallowing-in-progress frames may be subjected to movement average processing corresponding to the time axis. For example, the weighting function f(n) of the n-th frame is subjected to simple movement average processing for the previous and subsequent 20 frames. That is, f(n) is calculated by f(n) = (f(n-9) + f(n-8) + ... + f(n+10))/20. For changing the type of the frame to the swallowing-in-progress frame or the swallowing-not-in-progress frame by the value of the weighting function, as in the frame type change unit 60, a type change threshold value (for example, 0.5) is used. The range of the movement average and the type change threshold value may be other values without being limited to these values, the calculation expression for the movement average is not limited to the simple movement average shown as an example, and a generally known movement average calculation method such as a central movement average, a backward movement average, a forward movement average, or a weighted movement average can also be used.

In the present embodiment, the hardware structures (control processor 62 or the like) of the processing units that perform various types of processing, such as the controller 30, the image acquisition unit 31, the display controller 32, the image acquisition unit 33, the swallowing determination unit 34, the result recording unit 35, the first frame classification unit 40, the swallowing block generation unit 41, the temporal width determination unit 42, the second frame classification unit 50, the motion-specific swallowing block generation unit 51, the temporal width determination unit 52, the expression pattern determination unit 53, the third frame classification unit 55, and the fourth frame classification unit 56 (not shown), are various processors as shown below. The various processors include a central processing unit (CPU) that is a general-purpose processor that executes software (programs) to function as various processing units, a graphics processing unit (GPU) that execute image processing at high speed, a programmable logic device (PLD) that is a processor capable of changing a circuit configuration after manufacture, such as a field programmable gate array (FPGA), and an exclusive electric circuit that is a processor having a circuit configuration exclusively designed to execute various types of processing.

One processing unit may be configured by one of these various processors, or may be configured by a combination of two or more same type or different type of processors (for example, a plurality of FPGAs, or a combination of a CPU and an FPGA). Also, a plurality of the processing units may be configured by one processor. As an example in which the plurality of processing units are configured by one processor, first, there is a form in which one processor is configured by a combination of one or more CPUs and software, and this processor functions as the plurality of processing units, as represented by a computer, such as a client or a server. Second, as represented by a system-on-chip (SoC) or the like, there is a form in which the processor is used in which the functions of the entire system which includes the plurality of processing units are realized by a single integrated circuit (IC) chip. In this way, various processing units are configured by one or more of the various processors described above, as the hardware structure.

Further, the hardware structure of these various processors is, more specifically, an electric circuit (circuitry) having a form in which circuit elements, such as semiconductor elements, are combined. In addition, the hardware structure of the storage unit is a storage device, such as a hard disc drive (HDD) or a solid-state drive (SSD).

### Explanation of References

10: endoscope system
12: endoscope
12a: insertion part
12b: operating part
12c: bendable part
12d: distal end part
12e: angle knob
12h: still image acquisition instruction switch
12i: zoom operation unit
14: light source device
15: processor device
16: computer
17: recording device
18: display
19: user interface
20: light source unit
22: light source controller
30: controller
31: image acquisition unit
32: display controller
33: image acquisition unit
34: swallowing determination unit
35: result recording unit
40: first frame classification unit
41: swallowing block generation unit
42: temporal width determination unit
50: second frame classification unit
51: motion-specific swallowing block generation unit
52: temporal width determination unit
53: expression pattern determination unit
55: third frame classification unit
56: fourth frame classification unit
60: frame type change unit
62: control processor
63: program memory
Es: esophagus
Eg: epiglottis
Ev: epiglottic vallecula
F: food
Rg: rima glottidis
Ps: pyriform sinus
Sp: soft palate
To: tongue
Tr: trachea

## Claims

1. An image processing device comprising:
a control processor,
wherein the control processor is configured to:
acquire an examination image;
classify each frame of the examination image into any one of a swallowing-in-progress frame in which swallowing is in progress or a swallowing-not-in-progress frame in which the swallowing is not in progress; and
integrate, in a case in which a temporal interval between the swallowing-in-progress frames temporally adjacent to each other is equal to or less than a first threshold value, at least the adjacent swallowing-in-progress frames as a swallowing block.

2. The image processing device according to claim 1,
wherein the control processor is configured to determine that the swallowing-in-progress frame of which a temporal width is equal to or less than a second threshold value or equal to or more than a third threshold value is not the swallowing-in-progress frame, or determine that the swallowing block of which a temporal width is equal to or less than the second threshold value or equal to or more than the third threshold value is not the swallowing block.

3. The image processing device according to claim 1,
wherein the control processor is configured to:
classify each frame of the examination image into any one of a plurality of swallowing motion-specific frames in accordance with swallowing motion or the swallowing-not-in-progress frame; and
integrate, in a case in which a temporal interval between the swallowing motion-specific frames temporally adjacent to each other is equal to or less than the first threshold value, at least the adjacent swallowing motion-specific frames as a motion-specific swallowing block.

4. The image processing device according to claim 3,
wherein the control processor is configured to determine that the swallowing motion-specific frame of which a temporal width is equal to or less than a second threshold value or equal to or more than a third threshold value is not the swallowing motion-specific frame, or determine that the motion-specific swallowing block of which a temporal width is equal to or less than the second threshold value or equal to or more than the third threshold value is not the motion-specific swallowing block.

5. The image processing device according to claim 3,
wherein the control processor is configured to determine, based on an expression pattern including the swallowing motion-specific frame or the motion-specific swallowing block, whether the expression pattern indicates that the swallowing is in progress or that the swallowing is not in progress.

6. The image processing device according to claim 3,
wherein the swallowing motion-specific frame includes a pre-swallowing frame, a swallowing-in-progress frame, or a post-swallowing frame.

7. The image processing device according to claim 6,
wherein the pre-swallowing frame includes a pre-swallowing blur frame, an epiglottis inversion frame, or a food intake frame.

8. The image processing device according to claim 6,
wherein the swallowing-in-progress frame includes an overexposure frame, a shielding frame, a flow frame, a colored water inflow frame, or a swallowing food frame.

9. The image processing device according to claim 6,
wherein the post-swallowing frame includes a post-swallowing blur frame or a blackout frame.

10. The image processing device according to claim 3,
wherein, in a case in which the control processor is configured to classify the examination image into a plurality of lightness information frames in accordance with lightness,
the control processor is configured to determine, based on an expression pattern including the lightness information frame, the swallowing motion-specific frame, or the motion-specific swallowing block, whether the expression pattern indicates that the swallowing is in progress or that the swallowing is not in progress.

11. The image processing device according to claim 10,
wherein the lightness information frame includes a high-lightness frame, a medium-lightness frame, or a low-lightness frame.

12. The image processing device according to claim 3,
wherein, in a case in which the control processor is configured to classify a frame in which a blur of the examination image is equal to or less than a blur threshold value, as a pre-swallowing blur frame,
the control processor is configured to determine, based on an expression pattern including the pre-swallowing blur frame, the swallowing motion-specific frame, or the motion-specific swallowing block, whether the expression pattern indicates that the swallowing is in progress or that the swallowing is not in progress.

13. The image processing device according to claim 1,
wherein the control processor is configured to calculate, for each frame, a value of a weighting function for evaluating whether the swallowing is in progress or the swallowing is not in progress, and change a type of the frame to the swallowing-in-progress frame or the swallowing-not-in-progress frame based on the value of the weighting function.

14. An operation method of an image processing device, the operation method comprising:
causing a control processor to execute:
acquiring an examination image;
classifying each frame of the examination image into any one of a swallowing-in-progress frame in which swallowing is in progress or a swallowing-not-in-progress frame in which the swallowing is not in progress; and
integrating, in a case in which a temporal interval between the swallowing-in-progress frames temporally adjacent to each other is equal to or less than a first threshold value, at least the adjacent swallowing-in-progress frames as a swallowing block.

15. The operation method of an image processing device according to claim 14,
wherein the control processor is caused to determine that the swallowing-in-progress frame of which a temporal width is equal to or less than a second threshold value or equal to or more than a third threshold value is not the swallowing-in-progress frame, or determine that the swallowing block of which a temporal width is equal to or less than the second threshold value or equal to or more than the third threshold value is not the swallowing block.
